# EUROPEAN PATENT APPLICATION

(11) **EP 4 282 850 A1**
(43) Date of publication of application: **29.11.2023**
(21) Application number: 23174435.0
(22) Date of filing: 22.05.2023
(51) Int. Cl.: C05F 1/00, B09B 3/35, B09B 3/40, B09B 3/60, C05F 3/00, C05F 5/00, C05F 17/20, C12M 1/33, C12M 1/00, C05F 17/60

(54) **PROCESSING ORGANIC WASTE**

(30) Priority: 24.05.2022 GB 202207574
(71) Applicant: Lohas Recycling Limited, Oswestry Wales SY10 8BP (GB)
(72) Inventor: Kao, Ihsien, Oswestry, SY10 8BP (GB)
(74) Representative: Strachan, Victoria Jane

(57) **Abstract**

A method of processing raw organic waste, comprising the steps of:
- pre-processing said organic waste to bring its moisture content to 45-70%wt (100 - 104);
- obtaining a batch of said pre-processed organic waste and adding a quantity of dry organic particulate or granular material and at least 2%wt of digestive enzymes thereto to produce an organic waste mix for processing (110);
and, subsequently,
- processing said organic waste mix by heating it to substantially 80°C and maintaining it at substantially 80°C for at least 1 hour, whilst continuously mixing said organic waste mix (112).

## Description

### Field of the Invention

This invention relates generally to a method of processing organic waste, apparatus for processing organic waste, and a fertilizer produced by processing organic waste, that is suitable for organic growing.

### Background of the Invention

In recent years, there has been an ongoing drive toward the use of organic fertilizers in agriculture, rather than synthetic chemical fertilizer compositions. Compost is a well known organic fertilizer that is commonly prepared by decomposing plant and food waste and recycling organic materials. Composting is a natural process that stems through microbial succession, marking the degradation and stabilization of organic matter present in such waste. In most cases, the composting process can be broken into two stages: a first, fermentation stage, and a subsequent, second maturing stage during which any residual organic matter that is more difficult to decompose is further decomposed and stabilized.

However, traditional methods of preparing organic fertilizer from organic waste by microbial composting have a number of drawbacks associated with them, especially when considered for large-scale agricultural use.

Firstly, the complete composting process can take a significant period of time, often three to six months, to complete, and a very large space is required to prepare compost in any great quantities. Secondly, there is often a significant loss (50% or more) of nitrogen from the mixture during the microbial composting process, which is clearly undesirable since nitrogen is one of the key nutrients required in a fertilizer. Furthermore, the environmental impact of the microbial composting process is significant in terms of carbon emissions, waste water and strong odours. Finally, using traditional microbial composting techniques, it can be very difficult, if not impossible, to maintain the organic waste mixture at a sufficiently high temperature for a long enough period of time to eliminate pathogenic bacteria. As a result, the resulting fertilizer is highly likely to contain at least some undesirable pathogenic bacteria.

It is an object of aspects of the present invention to address at least one or more of the above-mentioned issues.

### Statements of Invention

In accordance with a first aspect of the present invention, there is provided method of processing a batch of raw organic waste, comprising the steps of:
- pre-processing said batch of organic waste to bring its moisture content to 45-70%wt;
- adding a quantity of dry organic particulate or granular material and at least 2%wt of digestive enzymes to said pre-processed batch of organic waste to produce an organic waste mix for processing;
and, subsequently,
- processing said organic waste mix by heating it to substantially 80°C and maintaining it at substantially 80°C for at least 1 hour, whilst continuously mixing said organic waste mix.

In a preferred embodiment, said digestive enzymes may comprise a neutral protease.

Beneficially, said quantity of dry organic particulate or granular material may be substantially 10%wt.

In an embodiment, said pre-processing step may include determining a moisture content of said batch of raw organic waste and either partially dewatering it or adding liquid thereto to bring said moisture content to around 50%wt (beneficially, ~45 - 55%wt). In this case, the quantity of digestive enzymes added to the batch may, beneficially, be 2%wt.

In the case of some types of raw organic waste, for example, uncooked food waste, the method may include an additional pre-processing step comprising crushing said raw organic waste. Beneficially, said crushing step, if performed, comprises reducing the size of solid pieces of organic waste to substantially 1cm or less.

Beneficially, said organic waste mix may be maintained at substantially 80°C for substantially 1 hour, and then caused/allowed to cool for a further period of 1 - 1.5 hours, whilst continuously mixing said organic waste mix..

Optionally, the method may further comprise the step of weighing a said batch of said pre-processed organic waste prior to adding said enzymes and dry organic particulate or granular material thereto.

By way of example, the dry organic particulate or granular material may comprise one or more of sawdust, bran, husk.

It will be appreciated that, beneficially, the digestive enzyme added to the batch of pre-processed organic waste may be selected according to the type of the raw organic waste, which may comprise one of poultry manure, broiler manure, pig manure, cow/horse/sheep manure, raw food waste, cooked food waste, plant material, animal feathers, or animal remains.

Another aspect of the invention extends to a fertilizer produced by the method substantially as described above.

These and other aspects of the invention will be apparent from the following detailed description.

### Brief Description of the Drawings

Embodiments of the present invention will now be described, by way of examples only, and with reference to the accompanying drawings, in which:
Figure 1 is a flow diagram illustrating a method according to an exemplary embodiment of the invention for processing organic waste; and
Figure 2 is a schematic diagram illustrating an apparatus when used in a method according to an exemplary embodiment of the invention for processing organic waste.

### Detailed Description

In a method according to an exemplary embodiment of the present invention for processing organic waste, the organic waste is subjected to two stages: a pre-processing stage and a fermentation stage.

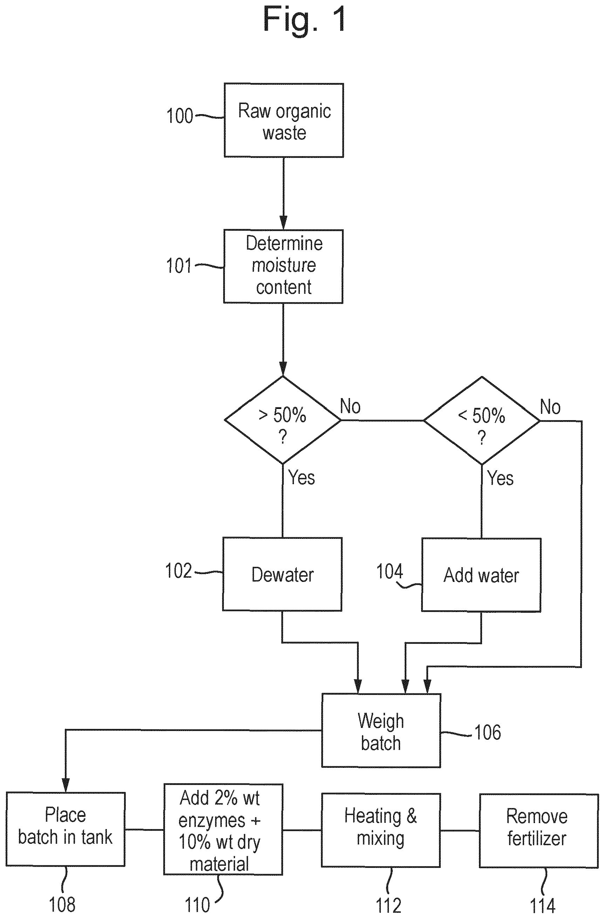

Referring to Figure 1 of the drawings, in a first step 100 a quantity of raw organic waste is obtained and, if necessary, crushed to create a mixture containing small pieces of organic waste and water. This crushing step is only necessary for some types of organic waste, such as uncooked food waste for example, because some of the solid pieces of waste may be relatively large and uncooked food is relatively more difficult to decompose during the fermentation stage. Other types of organic waste, such as chicken manure, do not need to be crushed, as the particulate matter is already sufficiently small and relatively easy to decompose during the fermentation stage. It will be apparent that, the smaller the pieces of solid organic waste, the greater will be the surface area of the waste over which the enzymes used during the fermentation stage will pass and, therefore, the more efficient the fermentation process will be. Ideally, the pieces of solid organic waste should be <~1 cm in order to ensure that the surface area of the waste over which the enzymes pass is sufficient to ensure the desired efficiency and efficacy of the fermentation process.

In a pre-processing step, the moisture content of the organic waste mixture is determined and, if required, increased or reduced. Ideally, the moisture content of the organic waste mixture delivered to the main processing phase should be substantially 45-55%wt, and, beneficially, close to ~50%wt. The digestive enzymes added (hereinafter, at step 110) need water to be activated and water helps to ensure that the enzymes contact all of the organic waste during processing, to ensure that large molecules, such as proteins, are broken down. At around 45%wt water/moisture, ~2%wt of digestive enzymes starts to work efficiently and ~50%wt water is considered to be the most steady and economic moisture content. If the moisture content is between, say, 55%wt and 70%wt, the process will still work, but a greater quantity of digestive enzymes is required, which can greatly increase the cost. At greater than 70%wt moisture content, the processing phase may not be wholly successful, irrespective of the amount of enzymes utilized and, accordingly, dewatering is required to reduce the moisture content.

Accordingly, using a moisture meter, the moisture content of the organic waste mixture is determined at step 101. If the moisture content is determined to be greater than 45-55%wt (ideally at or close to 50%wt), the raw organic waste is dewatered at step 102. Dewatering of organic waste is a known process (largely intended to reduce its weight, reduce transport costs and emissions, and prevent environmentally harmful leakage). In a known dewatering process, the raw organic waste may, for example, be placed in a screw press (or similar apparatus) and subjected to a gradual increase in pressure as it moves through the press to the exit, where liquid is forced out through a screen mesh. However, in a conventional dewatering process, the object is to remove as much moisture from the raw organic waste as possible, whereas in the present method, the object of the dewatering pre-processing step is to reduce the moisture content to around 50%wt.

If, on the other hand, it is determined at step 100 that the moisture content of the raw organic waste is less than 50%wt, water is added to the waste (at step 104) until the moisture content is around 50%wt.

A batch of the pre-processed raw organic waste is then weighed out at step 106, ready to be placed into fermentation apparatus for the fermentation stage of the method. The weight of a batch is determined according to the volume of material that can be processed by the fermentation apparatus, which will vary. For example, batches of 2, 3, 5 or 8 tons of pre-processed organic waste can be processed by the fermentation apparatus, according to the size of the tank thereof.

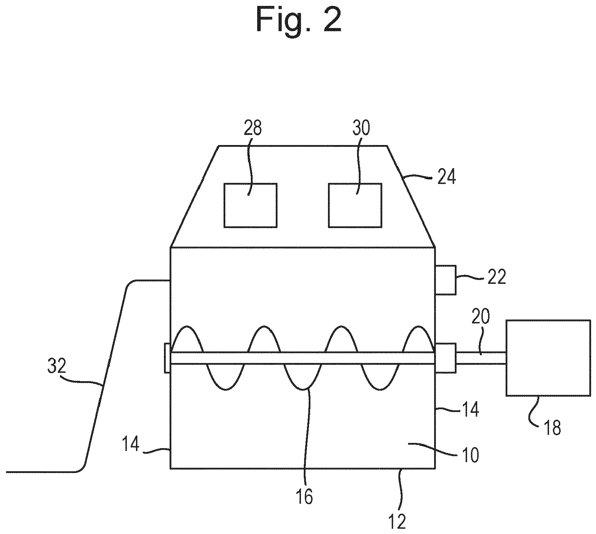

Referring additionally to Figure 2 of the drawings, fermentation apparatus for use in a method according to an exemplary embodiment of the present invention comprises a large tank 10 having a base 12, side walls, and opposing end walls 14. An elongate spiral mixer 16 extends through the tank 10, between the opposing end walls 14. A drive motor 18 is mounted adjacent the outer surface of one of the end walls, and its shaft 20 is coupled to one end of the spiral mixer 16. The drive motor 18 is configured, in use, to rotate the spiral mixer 16 within the tank 10. A fan 22 is mounted outside the tank 10, on or adjacent to one of the side or end walls, toward the top of the tank 10 (when oriented for use, with the base 12 at the bottom), and arranged and configured to blow air into the volume of the tank 10. The fan 22 is used to expel steam from inside the tank 10 during processing and can contribute to reducing moisture. The fan 22 is also used to control and adjust air flow volume within the tank 10, because too much air flow volume will cause an undesirable temperature reduction, so a control unit acts to balance the need to expel steam whilst ensuring that the temperature is maintained by controlling air flow volume within the tank 10.

At the top of the tank 10, opposite the base 12, there is provided a cover 24 comprising a pair of side walls, extending at an angle from respective side walls of the tank 10 and meeting to form an apex, and a pair of opposing triangular end walls creating closed 'roof'-like structure over the tank 10. One of the side walls of the cover 24 is provided with a pair of openings covered with respective hatches 28, 30 or flaps that can be selectively opened and closed/sealed. The 'roof'-like structure of the cover 24 acts as a drainage system, wherein steam condenses on the inner surface of the cover 24 and the resultant water drains out of the tank via a side gutter.

A conveyor 32 extends from an opening in one of the side or end walls of the tank 10 and is arranged and configured to carry material into the tank for processing.

At step 108 of the method, a weighed batch of pre-processed raw organic waste is carried into the tank 10, via the conveyor 32. The weight/volume of the batch is such that its level within the tank, 10, once loaded, fills, at most, around 90% of the volume of the tank 10, such that it can be evenly mixed without overflowing.

Next, at step 110, a quantity of enzymes and a quantity of loose dry material is added to the batch via the hatches 28, 30. The enzymes chosen will be dependent on the type of organic waste to be processed, as will be described in more detail hereinafter. The dry material, which is also preferably organic, could, for example, comprise sawdust, husk, bran, etc., and its purpose is balance the texture of the material and make it soft and loose during mixing, so as to increase enzyme activity and increase their efficiency and effectiveness in breaking down the solid organic waste matter.

The quantities of enzymes and dry materials added to the batch of organic waste are dependent on the size of the batch and are determined by the weight thereof. In the present method, around 2%wt of enzymes (in powder form) is added and 10%wt of dry materials is added via the hatches 28, 30. If the quantity of digestive enzymes added is less than 2%wt, it is highly possible that the complete batch will not fully ferment during the fermentation process. More than 2%wt can, of course, be used, but that greatly increases the cost of the process. One of the key advantages of the proposed method is that, by control of the moisture content of the material delivered to the fermentation process, as little as 2%wt of enzymes can be used to fully ferment a batch of organic waste material in a total of approximately 3 hours.

At step 112, the motor 18 is switched on so as to start rotating the spiral mixer 16 so as to mix the batch now comprised of the pre-processed organic waste, the enzymes and the dry materials, and a heating means (not shown) is switched on to start heating the inner volume of the tank 10. The heating means may, for example, be an indirect heating means comprising thermal oil in contact with with the wall of the tank 10. The thermal oil may be heated using an electric element or an external thermal oil boiler may be used, for example. The spiral mixer 16 will continue mixing throughout the fermentation stage, which is composed of two phases. In a first phase, the temperature in the tank 10 rises, thereby raising the temperature of the batch. A temperature sensor (not shown) monitors the temperature of the batch. The first, heating phase continues until the temperature of the batch is at or around 80°C. The time this takes will be dependent on the temperature of the environment in which the apparatus is operating, but might typically be 30 - 50 minutes. Subsequently, in a second phase, the temperature of the batch is maintained at or around 80°C be a control system that receives temperature data from the temperature sensor and switches the heating means on and off as required, as well as utilizing the fan 22, to maintain the temperature of the batch at ~80°C and blow steam out of the tank (as described above). The temperature of the batch is so maintained at ~80°C, and continuously mixed, for approximately 60 minutes. After that, the mixing is (continuously) continued for a further 1 - 1.5 hours, but the heat is switched off and the fan 22 continues to blow steam out of the tank 10 to cool the mixture. After that, the batch is ready to be unloaded, giving a total fermentation time of no more than around 3 hours until the enzymes have broken down the organic matter completely.

Once the fermentation stage is complete, the contents of the tank 10 can be removed (at step 114). The contents output from the fermentation stage is a safe and stable fertilizer that is pasteurised and safe for use in organic agricultural growing. The nutrients from the organic waste are retained and, in some exemplary embodiments at least, an N (Nitrogen)-P (Phosphorous)-K(Potassium) ratio of 4-1-2 can be achieved.

In an exemplary embodiment, the general term for digestive enzymes suitable for use in the method described above is Neutral Proteases. A different specific Neutral Protease is required for different classes of organic waste. Such enzymes are available ready for use from various manufacturers and the names given to the enzymes varies according to the manufacturer thereof. In an exemplary embodiment, enzymes from, for example, Tetanti AgriBiotech Inc. may be utilized, as set out in the table below:

| **Types of enzymes** | **TTT number** |
|---|---|
| **1. Poultry manure:** | |
| □**For laying hen manure** | 1-1 |
| □**Special for broiler manure** | 1-2 |
| □**Special for pig manure** | 1-3 |
| □**For cattle, horses and sheep manure** | 1-4 |
| □**Other feces only:______** | 1-5 |

| **2. Food waste:** | |
|---|---|
| □**Special for raw kitchen waste** | 2-1 |
| □ **Dedicated for cooked food waste** | 2-2 |

| **3. Plants:** | |
|---|---|
| □**Special for straw, grain shell, branches and leaves, bagasse** | 3-1 |
| □**Special for oil palm and coconut residue fiber** | 3-2 |
| □**Special for sawdust and mushroom bag** | 3-3 |
| □**Special for plant residue ( dregs, distiller's grains )** | 3-4 |

| **4. Sludge:** | |
|---|---|
| □**Special for alkaline sludge** | 4-1 |
| □**Special for medium and acid sludge** | 4-2 |

| **5. animal:** | |
|---|---|
| □**Special for feathers** | 5-1 |
| □**Special for animal remains and internal organs** | 5-2 |

| **6. other kind:** | |
|---|---|
| □**Special for traditional Chinese medicine dregs** | 6-1 |
| □**Special for antibiotic bacteria residue** | 6-2 |
| □**Special for liquid fertilizer enzymes** | 6-3 |

Whilst the enzymes used may vary according to the organic waste that is being processed, the process, as described above, remains the same for all and results in a safe and stable fertilizer that is suitable for organic growing.

It will be apparent to a person skilled in the art, from the foregoing description, that modifications and variations can be made to the described embodiments without departing from the scope of the invention as defined by the appended claims.

## Claims

**1.** A method of processing raw organic waste, comprising the steps of:
- pre-processing said organic waste to bring its moisture content to 45-70%wt;
- obtaining a batch of said pre-processed organic waste and adding a quantity of dry organic particulate or granular material and at least 2%wt of digestive enzymes thereto to produce an organic waste mix for processing;
and, subsequently,
- processing said organic waste mix by heating it to substantially 80°C and maintaining it at substantially 80°C for at least 1 hour, whilst continuously mixing said organic waste mix.

**2.** A method according to claim 1, wherein said digestive enzymes comprise a neutral protease.

**3.** A method according to claim 1 or claim 2, wherein said quantity of dry organic particulate or granular material is substantially 10%wt.

**4.** A method according to any of the preceding claims, wherein said pre-processing step includes determining a moisture content of said batch of raw organic waste and either partially dewatering it or adding liquid thereto to bring said moisture content to substantially 45-55%wt.

**5.** A method according to claim 4, wherein said pre-processing step comprises bringing said moisture content to substantially 50%wt.

**6.** A method according to claim 4 or claim 5, wherein substantially 2%wt of digestive enzymes is added to said batch.

**7.** A method according to any of the preceding claims, including an additional pre-processing step comprising crushing said raw organic waste.

**8.** A method according to claim 7, wherein said crushing step comprises reducing the size of solid pieces of raw organic waste to less than ~1cm.

**9.** A method according to any of the preceding claims, wherein said organic waste mix is maintained at substantially 80°C for substantially 1 hour, and then allowed/caused to cool for a further 1 - 1.5 hours, whilst continuously mixing said organic waste mix.

**19.** A method according to any of the preceding claims, wherein the dry organic particulate or granular material comprises one or more of sawdust, bran, husk.

**11.** A method according to any of the preceding claims, further comprising the step of weighing a said batch of said pre-processed organic waste prior to adding said enzymes and dry organic particulate or granular material thereto.

**12.** A method according to any of the preceding claims, wherein said digestive enzyme is selected according to the type of the raw organic waste.

**13.** A method according to any of the preceding claims, wherein the raw organic waste comprises one of poultry manure, broiler manure, pig manure, cow/horse/sheep manure, raw food waste, cooked food waste, plant material, animal feathers, or animal remains.

**14.** A fertilizer produced by the method of any of claims 1 to 13.
